# DEMANDE DE BREVET EUROPEEN

(11) **EP 1 435 219 A1**
(43) Date de publication de la demande: **07.07.2004**
(21) Numéro de dépôt: 02080684.0
(22) Date de dépôt: 31.12.2002
(51) Int. Cl.: A61B 17/02

(54) **Ecarteur chirurgical stéréotaxique**

(71) Demandeur: Deldime, Paul, 6700 Arlon (BE)
(72) Inventeur: Deldime, Paul, 6700 Arlon (BE)

(57) **Abrégé**

Ecarteur chirurgical stéréotaxique fabriqué en une matière rigide et médicalement acceptable pour réaliser une procédure chirurgicale sans l'aide d'un tiers spécialisé, caractérisé en ce qu'il comprend un anneau ou orbite équatorial **(11)**, une anse ou orbite méridionale **(13)**, un bras horizontal **(9)** se positionnant au-dessus ou à l'extérieur de n'importe quelle zone de l'anatomie humaine ou animale définie comme occupant une positon inférieure ou hémisphérique Sud, l'écarteur occupant une zone d'écartement hémisphérique Nord, supérieure ou externe par rapport au plan du sujet humain ou animal, cette zone d'écartement étant circonscrite par l'anneau ou orbite équatoriale **(11)** et une anse ou orbite méridionale **(13)** qui peuvent se mouvoir l'une par rapport à l'autre à des degrés et des inclinaisons variables. Des curseurs **(12)** et **(14)** adaptés à chacune des orbites fixent la rétraction de porteurs d'instruments chirurgicaux de base **(16)** ou des instrumentations spécifiques **(15)** vers n'importe quel point hémisphérique Sud entourant le champ opératoire **(1)**.

## Description

L'invention concerne un écarteur chirurgical stéréotaxique.

Un écarteur chirurgical est un instrument servant à écarter les lèvres d'une plaie.

La présente invention est relative à un écarteur chirurgical permettant au chirurgien de réaliser un grand nombre d'interventions chirurgicales sans l'assistance opératoire physique d'un ou de plusieurs tiers spécialisés.

Une variété innombrable d'écarteurs chirurgicaux qu'ils soient spécifiques à certaines techniques ou polyvalents, existent depuis des décennies et porte le nom de chirurgiens réputés mais ces écarteurs nécessitent toujours la présence indispensable d'un ou de plusieurs aides spécialisés pour réaliser une procédure chirurgicale.

L'exposition adéquate d'une région opératoire constitue plus de la moitié de la technique opératoire.

Le champ d'application de cet écarteur chirurgical couvre toutes les interventions chirurgicales sur l'anatomie humaine ou animale ne nécessitant pas des expositions de champs opératoires étendus ou trop profonds ou vidéo-assistées qui constituent de par leur fréquence élevée un volume d'activité important du travail des chirurgiens.

Il est important de préciser pour la compréhension des termes descriptifs utilisés par la suite que, par rapport à un être humain ou animal couché sur une table d'opération, l'écarteur stéréotaxique est positionné au-dessus d'une de ses zones anatomiques.

Plusieurs inventions ( à titre exemplatif et pour n'en citer que quelques unes: US 3,998,217, US 6,464,634, réalisent l'écartement de la plaie de manière variable traction variable dans un seul plan. Une autre invention tel que US 5,755,660 reste limitée à quasi un seul type de procédure ( la cholécystectomie) qui se pratique actuellement le plus souvent par technique vidéo-assistée. L'invention US 6,431,025 rajoute la possibilité d'une inclinaison lors de la rétraction; elle convient parfaitement pour les procédures chirurgicales étendues et profondes mais nécessitent toujours la présence de un ou plusieurs tiers pour assister l'opérateur principal.

L'objectif de cette invention est de résoudre le problème de l'assistance opératoire du chirurgien qui devient aléatoire étant donné la pénurie actuelle des médecins du secteur chirurgical et l'absence d'un personnel de nursing spécialisé dont le coût élevé n'est pas couvert valablement par un système de financement, qu'il soit prévu par la loi ou non.

Cet objectif ainsi que d'autres qui apparaîtront au vu de la description détaillée sont atteints par l'écarteur chirurgical de l'invention suivant la partie caractérisante de la revendication **1**.

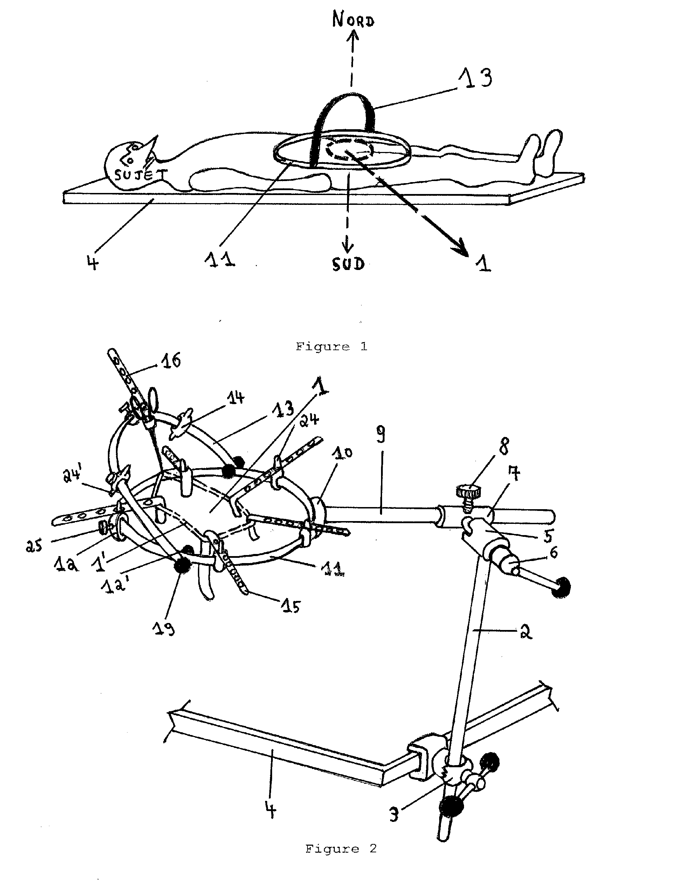

La partie essentielle de l'invention est la constitution d'un écarteur mécanique stéréotaxique permettant comme expliqué sur **la figure 1** l'exposition tridimensionnelle d'un champ opératoire **1** d'un sujet (humain ou animal) reposant sur une table d'opération **4** par un système de préhension et de traction d'un point anatomique quelconque de ce champ vers un degré variable d'une orbite méridionale **13** de direction Nord et d'une orbite équatoriale **11**.

Cet écarteur est défini comme occupant une zone hémisphérique Nord ou Supérieure par rapport au sujet (être humain ou animal) qui occupe une zone hémisphérique Sud ou Inférieure.

Cet écarteur s'adapte sur n'importe quel porteur mécanique solidaire des tables chirurgicales pour être centré au dessus de tout champ opératoire s'étendant de l'extrémité craniale à l'extrémité caudale ou de la tête aux pieds de toutes les anatomies.

Il comporte des accessoires porteurs permettant l'exploitation illimitée de toutes les petites instrumentations chirurgicales qui font partie intégrante des techniques chirurgicales de base et des accessoires spécifiques à son champ d'application.

**D'autres détails et particularités de l'invention ressortiront de la description données ci-après,** à titre d'exemple non limitatif d'une forme de réalisation particulière d'un écarteur chirurgical stéréotaxique et de son instrumentation spécifique, suivant l'invention, avec référence aux dessins annexes:
La **figure 1** schématise et précise la définition générale de l'exposition tridimensionnelle d'un champ opératoire.
La **figure 2** est une vue schématique en perspective de l'écarteur stéréotaxique fixé sur le bord d'une table d'opération.
La **figure 3** décrit le montant vertical.
La **figure 4** décrit le bras horizontal avec son anneau équatorial.
La **figure 5** décrit en perspective les deux curseurs **12'** spécifiques à la fixation de l'anse méridionale
La **figure 6** décrit l'anse méridionale.
La **figure 7** décrit l'assemblage et l'action combinés de l'anneau équatorial et de l'anse méridionale décrits aux figures 4 et 6.
La **figure 8** décrit les curseurs de l'anneau équatorial.
La **figure 9** décrit les curseurs de l'anse méridionale.
La **figure 10** décrit le porteur d'instrumentation.
La **figure 11** décrit à titre exemplatif, en perspective de face et de profil, une instrumentation spécifique de rétraction et d'accrochage, non limitative.
La **figure 12** décrit en vue de profil son placement sur un curseur équatorial.

L'écarteur stéréotaxique dénommé **"R.O.S.A."** (**R**etractor for **O**ne **S**urgeon **A**lone), suivant une forme de réalisation préférée de l'invention comme décrit dans la figure **2** représentant son assemblage général, comprend un bras horizontal tubulaire **9** dont l'une des extrémités es fermée et dont l'extrémité opposée est solidaire d'un support **10** permettant la fixation amovible d'un anneau **11** défini comme orbite équatoriale. L'ensemble prend par exemple la forme générale d'une raquette de tennis. Ce bras horizontal est fixé fermement sur un des bords latéraux **4** de n'importe quelle table chirurgicale par l'intermédiaire d'un montant vertical **2** réglable en hauteur, en rotation et en inclinaison.

Le bras horizontal **9** est enfilé dans un manchon **7** solidaire de l'extrémité supérieure du montant vertical qui comporte une poignée de serrage **6** permettant la rotation et la fixation du bras horizontal à n'importe quel point de sa longueur.

Sur l'anneau équatorial **11** sont enfilés un nombre variable de curseurs **12** coulissants librement. La forme de ces curseurs est de préférence un parallélépipède rectangle aux arrêtes arrondies, dont la partie centrale est rainurée pour être enfilée au plus juste tout en permettant son coulissement sur l'anneau équatorial. Avantageusement la section de l'anneau équatorial est de section rectangulaire à coins arrondis correspondant parfaitement à la rainure centrale du curseur. Chaque curseur comporte une vis **25** moletée de fixation externe permettant le blocage à un degré quelconque de l'anneau équatorial. Chaque curseur est prolongé à son extrémité supérieure par un doigt vertical **24** permettant l'accrochage d'un porteur d'instrumentation **16** ou d'un instrument spécifique **15**.

Le manche du porteur d'instrumentation **16** ou de l'instrument spécifique **15** est perforé à intervalle régulier pour autoriser une traction variable de l'instrumentation selon son accrochage sur le doigt vertical de chaque curseur. Suivant la forme de réalisation particulièrement avantageuse des alésages situés sur le manche d'une instrumentation spécifique **15**, la traction vers l'orbite équatoriale confère à l'instrument une inclinaison de degré variable de direction Sud permettant l'accrochage et la rétraction particulièrement efficace de n'importe quelle structure anatomique d'un champ opératoire vers un degré équatorial quelconque.

Deux curseurs **12'** de réalisation particulière et placés en opposition variable sur l'anneau équatorial permettent la fixation rapide de - 90° à +90° d'une anse **13** de la même section que l'anneau équatorial mais d'un rayon légèrement plus grand. Cette anse constitue l'orbite méridionale et peut pivoter librement selon un axe Nord-Sud sur l'anneau équatorial, à gauche ou à droite du point d'attache du bras horizontal.

Sur cette anse méridionale **13** peut être enfilé un nombre variable de curseurs **14** identiques aux curseurs équatoriaux **12** mais comportant deux doigts **24** et **24'** en opposition pour multiplier les possibilités de fixation de l'instrumentation en fonction de l'inclinaison de l'anse méridionale selon que le chirurgien travaille à droite ou à gauche de la table d'opération et cela indépendamment du fait que la fixation du bras vertical soit sur le bord droit ou gauche de la table.

**En conclusion,** cette instrumentation préférée de l'invention comprend entre autres et à titre principal les instruments suivants ( voir figure **2** ):
a) un montant vertical rigide de section ronde **2** dont l'extrémité supérieure se termine par une attache de serrage orientable **5** permettant d'enfiler
b) un bras horizontal **9** prolongé d'un anneau équatorial **11** de la forme semblable à une raquette de tennis,
c) une mâchoire indexable **3** amovible et orientable permettant la fixation de ce bras à une table d'opération chirurgicale **4**,
d) une anse méridionale **13** pivotant sur le pourtour de l'anneau équatorial **11** par l'intermédiaire de deux curseurs **12'** de réalisation particulière autorisant son inclinaison de -90° à +90°,
e) de curseurs **12** enfilés sur l'anneau équatorial et de curseurs **14** enfilés sur l'anse méridionale permettant la fixation
f) de porteurs **16** de petites instrumentations traditionnelles de forme et de dimension variables conçus pour écarter et rétracter un élément anatomique de la zone opératoire,
g) et/ou d'une instrumentation spécifique **15** dont la la réalisation permet une rétraction et une inclinaison de direction Sud.

**Forme, matière et mode d'utilisation:** La forme de réalisation particulière de l'écarteur stéréotaxique, suivant l'invention, telle que représentée sur les **figures 1 à 12** pour effectuer une procédure chirurgicale sans l'aide opératoire physique d'un ou de plusieurs tiers est réalisée en une matière rigide, médicalement acceptable, qui supporte sans se dégrader les différents modes de stérilisation permettant une asepsie rigoureuse lors de son utilisation. Pour ce faire, ses surfaces sont polies et toutes ses aspérités sont arrondies et polies.

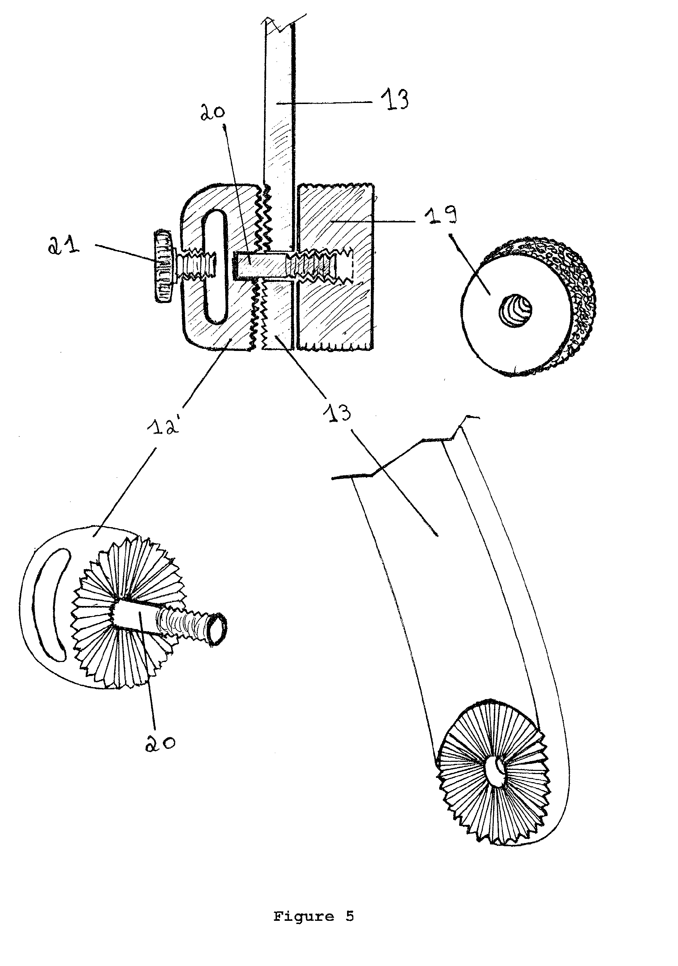

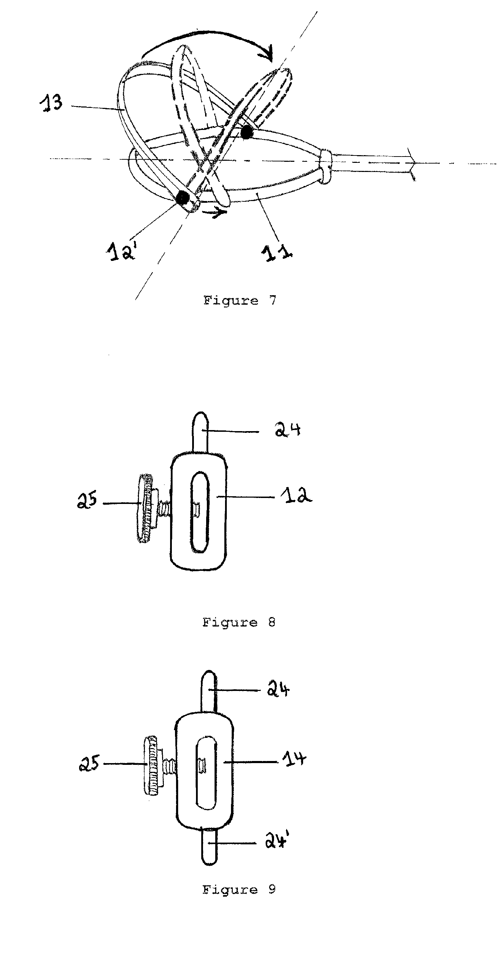

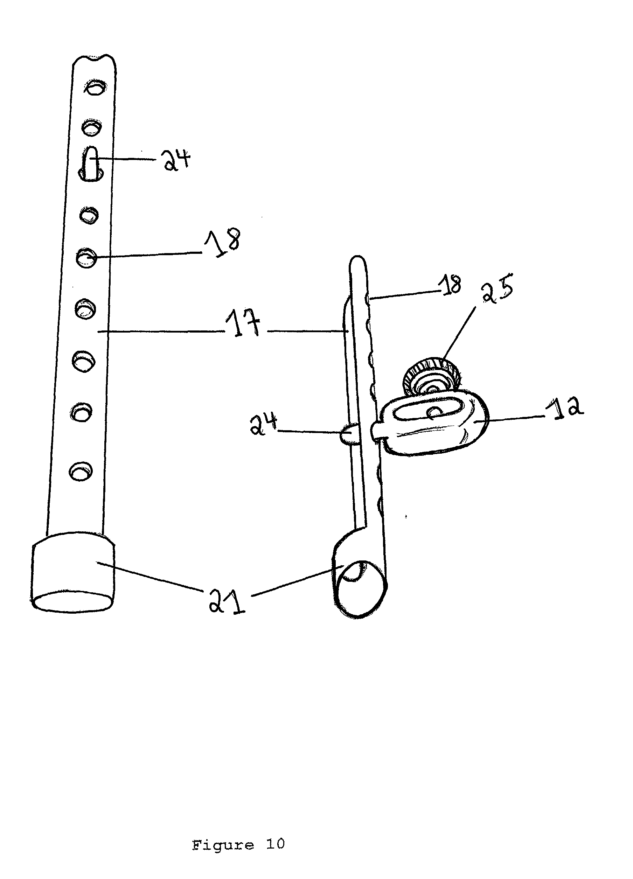

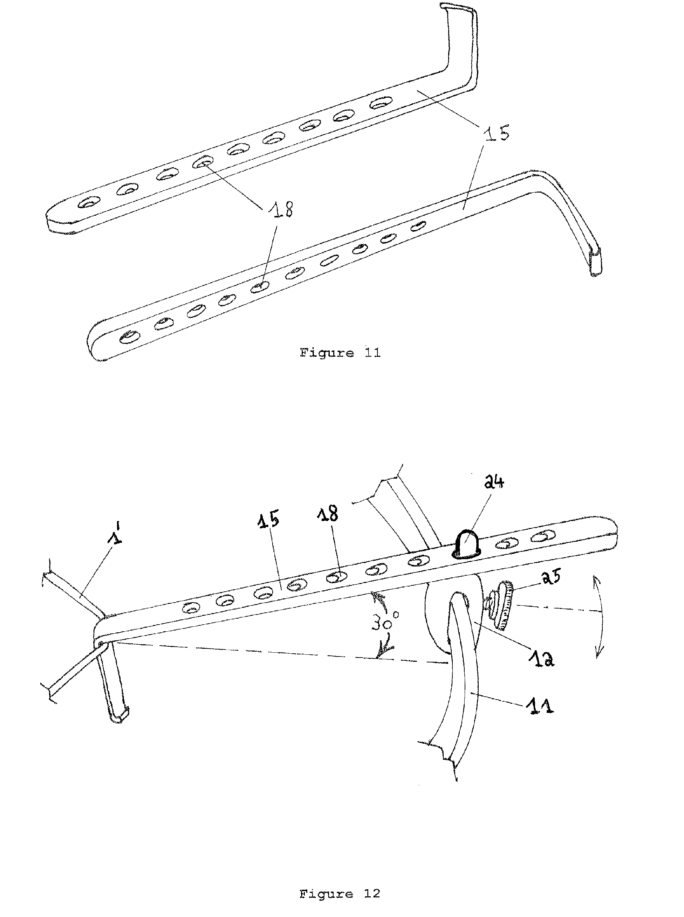

Comme montré clairement sur **la figure 2**, l'écarteur stéréotaxique comporte un montant vertical **2** constitué d'une barre ronde pleine dont la portion inférieure coulisse dans une mâchoire indexable **3** dont la réalisation autorise une rotation de 360° du montant sur son axe et une inclinaison de 360° parallèle à la table d'opération **4**. L'étau de serrage permet ensuite une fixation ferme dans la position choisie. La mâchoire indexable **3** se fixe fermement sur n'importe quel bord de la table d'opération **4**. Ce montant vertical **2** est solidaire d'une attache supérieure **5** comportant un système de serrage **6** d'un manchon **7** solidaire d'un axe perpendiculaire se terminant par une rotule pleine incluse dans le système de serrage de l'attache **5** et qui peut tourner sur 360° tout en se mouvant sur le plan horizontal de 0° à 180°. Ce manchon **7** comporte une vis moletée de serrage **8** immobilisant le bras horizontal **9** à n'importe quel point de sa surface. Le bras horizontal **9** constitué d'un tube fermé à une extrémité, est solidaire à son autre extrémité d'un support **10** profilé pour fixer et détacher l'anneau équatorial **11** qui épouse une forme courbe circulaire. La longueur du bras horizontal **9** doit être suffisante pour être porté au-dessus de tout champ opératoire sans gêner l'opérateur tout en respectant les règles de résistance et de stabilité de l'anneau équatorial **11** et de l'anse méridionale **13**. Son encombrement ne peut dépasser l'enceinte nécessaire à sa stérilisation. Sur l'anneau équatorial **11** sont enfilés un nombre variable de curseurs **12** qui sont décrits sur la **figure 8**. Deux de ces curseurs **12'**, diamétralement opposés sur l'anneau équatorial **11**, sont d'une réalisation qui autorise une rotation de l'anse méridionale sur son axe Nord-Sud et une inclinaison variable de 0° à 180° par rapport au plan de l'anneau équatorial **11**. Ils servent à la fixation de l'anse méridionale **13** selon un degré d'inclinaison choisi. Ils sont décrits sur la **figures 5**. Sur cette anse **13** sont enfilés des curseurs **14** décrits sur la **figure 9**. Tous les curseurs **12** ou **14** sont réalisés pour accrocher directement un instrument spécifique **15**, à titre exemplatif et de conception variée, décrit sur la **figure 11** ou un porteur d'instrumentation **16** décrit sur la **figure 10**. Sur la **figure 7** sont décrits l'assemblage et l'action combinée de l'anneau équatorial **11** et de l'anse ou orbite méridionale **13.**
L'ensemble de l'écarteur, selon l'invention, permet l'exposition parfaite d'une zone opératoire **1** circonscrite schématiquement par un double trait interrompu **1'.** Selon l'invention, l'anneau équatorial **11** peut présenter une forme circulaire, elliptique, ou toute autre courbe fermée.

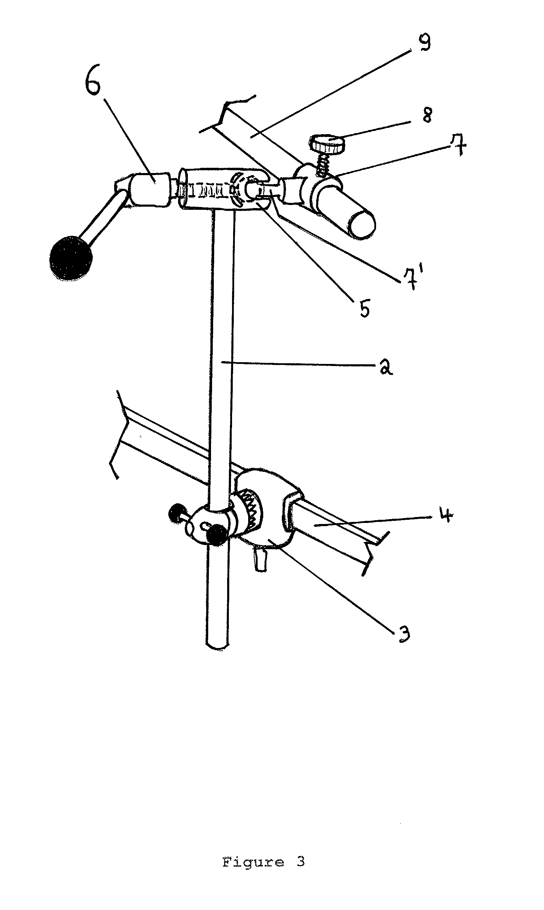

La **figure 3** décrit le montant vertical **2:** il est constitué d'une barre ronde **2,** pleine qui coulisse dans une mâchoire indexable **3,**fixée sur la table d'opération **4**, qui avant d'être serrée sur un point quelconque de sa surface, lui autorise une rotation de 360° sur son axe et une rotation de 360° parallèle à la table d'opération **4**. Son extrémité supérieure est solidaire d'un collier cylindrique de serrage **5** enfermant la rotule pleine que prolonge l'axe **7'** perpendiculaire au manchon **7** dans laquelle coulisse le bras horizontal **9**. La tête sphérique **7'** peut être bloquée dans une position choisie par une manette de serrage **6**. Cela permet au manchon **7** de tourner de 360° sur son axe et de se déplacer sur le plan horizontal de 0° à 180°. Une vis moletée de serrage **8** immobilise le bras horizontal **9** à un point quelconque de sa surface. L'ensemble du montant vertical permet donc le positionnement rapide et stable de l'écarteur stéréotaxique au-dessus de n'importe quel champ opératoire de l'anatomie humaine ou animale.

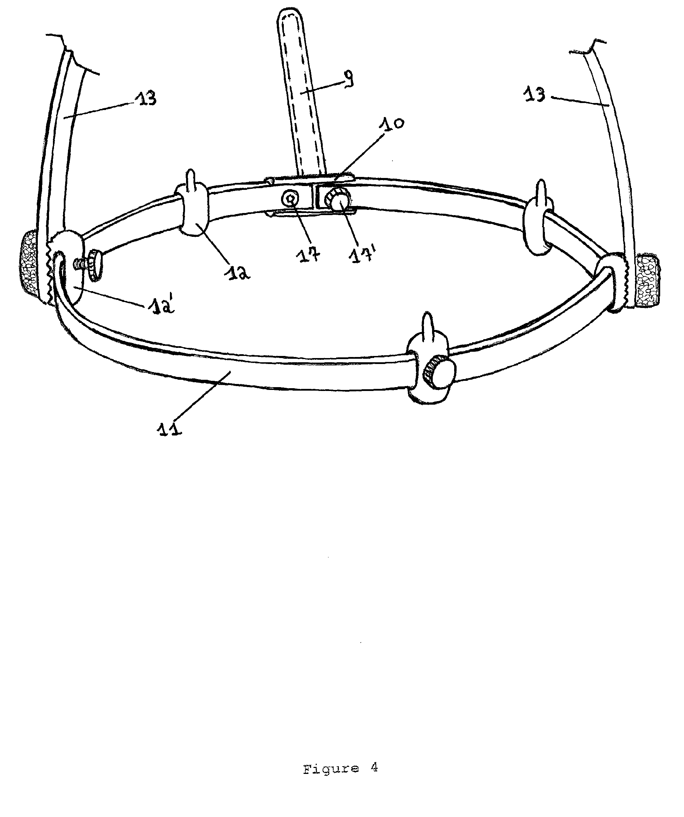

La **figure 4** décrit le bras horizontal **9** avec son anneau équatorial **11:** ce bras est constitué d'un tube **9** comportant un support **10** en forme de **T** terminant l'extrémité sur laquelle se fixe l'anneau équatorial **11** par une vis **17** ne dépassant pas la surface interne de l'anneau **11** et par une vis moletée **17'** autorisant le démontage facile pour rajouter un ou plusieurs curseurs **12**. L'anneau équatorial **11** peut être de dimension variable en épaisseur, largeur et longueur. Sur cet anneau équatorial **11** s'enfile en plus d'un nombre variable de curseurs **12**, deux curseurs **12'** particuliers, diamétralement opposés, dont la conception fixent l'anse méridionale **13** selon un diamètre quelconque du plan orbital équatorial tout en autorisant une inclinaison de 0° à 180° par rapport à l'anneau équatorial **11**. La longueur du bras **9** doit autoriser un positionnement très large de l'écarteur sur l'anatomie humaine ou animale tout en ne gênant pas le chirurgien.

En définitive le bras horizontal **9** avec son anneau équatorial attaché peut être comparé à un raquette de tennis.

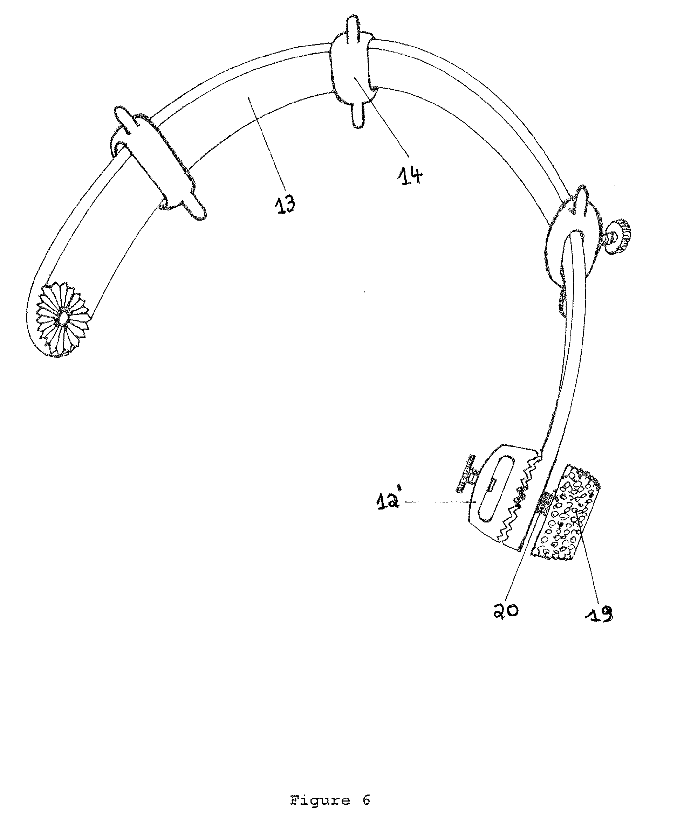

La **figure 5** décrit un des deux curseurs particuliers **12'** servant à manipuler et à fixer l'anse méridionale **13:** le curseur particulier **12'**, plus robuste, coulisse librement autour de l'anneau équatorial **11** non représenté sur la figure. L'anse méridionale **13** dont les deux extrémités de fixation comportent sur leurs faces interne des dispositifs d'engagement autobloquants, par exemple des stries radiales( voir aussi la **figure 6)** est imbriquée, lors du serrage de l'écrou moleté **19** sur la face externe du curseur **12'** qui comporte des dispositifs d'engagement autobloquants, par exemple des stries radiales, qui correspondent très exactement à ceux de la face interne des extrémités de fixation de l'anse méridionale **13**.
Cette imbrication des pièces **12', 13, 19** n'est possible que parce que un goujon central **20**, parfaitement solidaire et perpendiculaire à la face externe du curseur **12'** constitue l'axe central de cette imbrication. La longueur du goujon **20** et son extrémité filetée sur laquelle s'adapte l'écrou moleté **19** est réalisée de façon à serrer facilement et solidement les pièces **12',13,** et **19** selon une marge d'inclinaison choisie de 0° à 180°. Une vis moletée **21** fixe ensuite le curseur **12'** sur l'anneau équatorial **11** non représenté sur la figure.

La **figure 6** décrit l'anse méridionale **13:**
Elle est de même section que l'anneau équatorial **11** mais d'un rayon supérieur pour se fixer à l'extérieur via un curseur particulier **12'** décrit sur la **figure 5**. Un nombre indéterminé de curseurs **14** peuvent coulisser sur cette anse. Chaque extrémité de l'anse **13** comporte sur leur deux faces interne des stries radiales centrées sur l'axe du goujon **20** qui correspondent à la face externe du curseur particulier **12'** ce qui autorise le serrage ferme et rapide des pièces **12'** et **13** au moyen de l'écrou **19** comme décrit sur la **figure 5.**
La **figure 7** décrit l'assemblage et l'action combinée de l'anneau équatorial **11** et de l'anse méridionale **13:** L'anse méridionale **13** pivote sur son axe Nord-Sud autour de l'anneau équatorial **11** tout en ayant la possibilité de s'incliner de -90° à +90° par rapport à l'axe Nord-Sud grâce aux deux curseurs particuliers **12'** diamétralement opposés comme représentés sur la **figure 4.**
La **figure 8** décrit un des curseurs de l'anneau équatorial **11:**
Ces curseurs **12** sont de préférence des parallélépipèdes rectangles aux coins et arrêtes arrondies surmontés d'un doigt vertical supérieur **24** sur lequel s'adapte très exactement la petite instrumentation spécifique décrite en **figure 11** ou le porteur d'instrument **16**, de dimension et forme variable, décrit sur la **figure 10**. La face externe de ce parallélépipède est taraudée en son centre pour accepter une vis moletée de serrage **25** qui fixe le curseur **12** à un point quelconque de l'anse équatoriale **11**. En effet ce curseur est rainuré en son centre d'une manière telle qu'il entoure parfaitement la section transversale de l'anneau équatorial **11** pour autoriser son coulissement aisé tout en maintenant sa stabilité parfaite en position verticale.

La **figure 9** décrit un des curseurs **14** de l'anse méridionale **13:** c'est un curseur identique au curseur **12** mais il possède un doigt supplémentaire **24'** en opposition par rapport au doigt **24** ce qui permet de multiplier les possibilités de fixation et d'accrochage de porteur d'instrumentation **16** en fonction de l'inclinaison de l'anse méridionale **13** selon que le chirurgien travaille à droite ou à gauche de la table d'opération et cela indépendamment du fait que la fixation du bras vertical soit sur le bord droit ou gauche de la table d'opération.

La **figure 10** représente de face et de profil, à titre exemplatif, un porteur d'instrument **16** qui peut être de dimension variable: il est constitué d'un manche **17** de profil courbe et multi-perforé d'orifices **18** s'adaptant parfaitement autour des doigts **24.** Le manche **17** se termine par un anneau de forme variable **21** destiné à porter et à bloquer une instrumentation chirurgicale non spécifique lorsqu'il est rétracté à l'extérieur du champ opératoire.

La **figure 11** décrit, de face et de profil, à titre exemplatif une instrumentation spécifique **15** de rétraction et d'accrochage d'une structure anatomique quelconque: Le manche est multi-perforé à intervalle variable et régulier d'orifices **18** dont l'axe central est à 30° ( à titre exemplatif) par rapport au plan du manche de l'instrumentation ce qui confère une inclinaison identique à la pièce **15** lorsque qu'elle est placée en rétraction sur un des doigts **24** ( cfr. **figure 12**).

La **figure 12** décrit l'inclinaison particulière que prend une instrumentation spécifique **15** lorsqu'elle s'accroche sur le curseur **12:**
L'axe de perforation des orifices **18** forme un angle à titre exemplatif de 30° par rapport au manche de l'instrumentation **15** ce qui lui confère automatiquement une inclinaison correspondante de direction Sud lorsqu'elle est rétractée et accrochée sur le doigt vertical **24** du curseur **12.**

### Description des éléments présentés dans les figures 1 à 12 :

### Figure 1:

Définitions générales de l'exposition tridimensionnelle d'un champ opératoire d'un sujet:
**1**: champ opératoire ou zone de travail chirurgical
**4**: table d'opération
**11**: orbite équatoriale supérieure, latérale ou externe par rapport au sujet
**13**: orbite méridionale de direction Nord ou supérieure circonscrivant une zone hémisphérique Nord par rapport au sujet qui occupe une zone hémisphérique Sud ou inférieure.

### Figure 2:

Vue schématique de l'assemblage général de l'écarteur dénommé "**R.O.S.A.**" (**R**etractor for **O**ne **s**urgeon **A**lone):
**9:** bras horizontal tubulaire où manche de raquette
**10:** support solidaire de **9** et fermant une extrémité
**11:** anneau ou orbite équatoriale
**4:** bord latéral d'une table d'opération
**5:** dispositif de serrage
**2:** montant vertical
**7:** manchon solidaire du montant vertical **2**
**8:** vis moletée de serrage du bras **9** enfilé dans le manchon **7**
**6:** poignée de serrage du dispositif **5**
**12:** curseur coulissant sur l'anneau équatorial **11**
**25:** vis moletée fixant le curseur **12** sur l'anneau **11**
**24:** doigt vertical permettant l'accrochage
**16:** porteur d'instrumentation
**15:** instrumentation spécifique
**12':** curseur particulier fixant l'anse méridionale **13**
**13:** anse méridionale et orbite méridionale
**14:** curseur de l'anse méridional
**24':** doigt supplémentaire placé en opposition au doigt **24**

### Figure 3 :

Description du montant vertical **2:**
**2:** Montant vertical
**3:** Mâchoire indexable
**4:** table d'opération
**5:** collier cylindrique de serrage de la rotule de l'axe **7'**
**6:** manette de serrage de la rotule de l'axe **7'**
**7**: manchon de serrage dans laquelle s'enfile le bras **9**
**7'**:axe perpendiculaire avec sa rotule solidaire du manchon
**8**: vis moletée de serrage
**9:** bras horizontal

### Figure 4:

Description du bras horizontal avec son anneau équatorial
**9**: bras horizontal
**10**: support en T terminant une extrémité du bras **9**
**11**: anneau équatorial
**17**: vis de fixation de l'anneau équatorial **11** sur le support **10**
**17'**: vis moletée autorisant le démontage facile
**12:** curseur de l'anneau équatorial **11**
**12'**: curseur particulier de fixation de l'anse méridionale **13**
**13:** anse méridionale

### Figure 5:

Description en perspective d'un des deux curseurs particulier **12'** qui peuvent fixer de -90° à +90° l'anse méridionale **13** par rapport à l'anneau équatorial **11** non représenté.
**12':**curseur particulier enfilé sur l'anneau équatorial **11** non représenté
**13:** anse méridionale
**19:** écrou moleté
**20:** goujon central solidaire de **12'**
**21:** vis moletée de fixation du curseur **12'** sur l'anneau équatorial **11** non représenté

### Figure 6:

Description de l'anse méridionale **13:**
**13:** anse méridionale dont les extrémités de fixation comportent des stries radiales sur la face interne en concordance avec les stries radiales du curseur **12'**.
**14:** curseur de l'anse méridionale
**12':**curseur particulier de l'anneau équatorial **11** non représenté
**20:** goujon central solidaire et perpendiculaire au curseur **12'**
**19:** écrou moleté de serrage immobilisant les pièces **12' et 13**

### Figure 7:

Description de l'assemblage et de l'action combinée de l'anneau équatorial **11** et de l'anse méridionale **13**:
**13:** anse méridionale pivotant sur son axe Nord-Sud tout en ayant la possibilité de s'incliner de -90° à + 90° par rapport au plan de l'anneau équatorial **11.**
**11:** anneau équatorial
**12':** curseur particulier coulissant sur l'anneau équatorial **11** et fixant l'anse méridionale **13** comme décrit sur les **figures 4, 5, et 6.**

### Figure 8:

Description d'un des curseurs **12** de l'anneau équatorial **11:**
**12:** curseur s'enfilant sur l'anneau équatorial 11
**24:** doigt vertical supérieur
**25:** vis moletée de serrage fixant le curseur 12 sur l'anneau équatorial 11 non représenté.

### Figure 9:

Description d'un des curseurs **14** de l'anse méridionale **13** non représentée:
**14:** curseur s'enfilant sur l'anse méridionale **13**
**24':**doigt supplémentaire en opposition au doigt **24**
**25:** vis moletée de serrage du curseur **14** sur l'anse méridionale **13** non représentée.

### Figure 10:

Représentation de face et de profil, à titre exemplatif, d'un porteur d'instrument **16** variable en forme et dimension:
**17:** manche du porteur d'instrument de profil courbe à titre exemplatif multi-perforé d'orifice **18.**
**18:** orifice s'adaptant parfaitement pour être accroché sur le doigt **24** du curseur **12**
**21:** anneau de forme variable terminant une extrémité du manche **17** servant à porter et à bloquer une instrumentation chirurgicale non spécifique lorsqu'il est rétracté et accroché sur un des orifices **18** plus externe par rapport au champ opératoire

### Figure 11:

Description vue de face et de profil, à titre exemplatif une instrumentation spécifique **15** de rétraction et d'accrochage d'une structure anatomique quelconque:
**15**: instrumentation spécifique à titre exemplatif
**18:** orifices perforant le manche de l'instrumentation spécifique **15** à intervalle variable et régulier selon un axe central de 30° ( à titre exemplatif) par rapport son manche ( cfr. **figure 12** )

### Figure 12:

Description de l'inclinaison particulière que prend une instrumentation spécifique **15** ( à titre exemplatif) lorsqu'elle est accrochée et rétractée sur le curseur **12:**
**15**: instrumentation spécifique à titre exemplatif
**18:** un des orifices perforants le manche de l'instrumentation selon un axe de 30° ( à titre exemplatif) lui donnant une inclinaison de direction Sud identique en degré par rapport au plan de l'orbite équatoriale **11** lors de l'accrochage sur le doigt **24** du curseur **12.**
**12:** curseur de l'anneau ou orbite équatoriale **11**
**11:** anneau ou orbite équatoriale
**24:** doigt vertical du curseur **12.**
**25:** vis moletée de serrage immobilisant le curseur **12** sur l'anneau équatorial **11**.

Il est important de noter que l'invention n'est toutefois pas limitée aux formes de la réalisation particulière de certains de ses composants et accessoires telles que décrites ci-dessus et représentées aux dessins annexés, mais dans le cadre de l'invention, différentes variantes peuvent être envisagées.

## Revendications

1. Ecarteur chirurgical stéréotaxique fabriqué en une matière rigide et médicalement acceptable pour réaliser une procédure chirurgicale sans l'aide d'un tiers spécialisé, **caractérisé en ce qu'**il comprend un anneau ou orbite équatorial **(11)**, une anse ou orbite méridionale **(13)**, un bras horizontal **(9)** se positionnant au-dessus ou à l'extérieur de n'importe quelle zone de l'anatomie humaine ou animale définie comme occupant une positon inférieure ou hémisphérique Sud, l'écarteur occupant une zone d'écartement hémisphérique Nord, supérieure ou externe par rapport au plan du sujet humain ou animal, cette zone d'écartement étant circonscrite par l'anneau ou orbite équatoriale **(11)** et l'anse ou orbite méridionale **(13)** qui peuvent se mouvoir l'une par rapport à l'autre à des degrés et des inclinaisons variables.

2. Ecarteur suivant la revendication **1, caractérisé en ce que** chaque orbite équatoriale **(11)** et orbite méridionale **(13)** comporte un ou plusieurs curseurs **(12)** et **(14)** pouvant se mouvoir et se fixer à n'importe quel point des orbites précitées **(11 et 13)** pour permettre l'accrochage et la rétraction d'une instrumentation spécifique **(15)** ou d'un porteur d'instrumentation **(16)** destiné à saisir et à écarter vers un point quelconque hémisphérique Nord, supérieur, latéral ou externe une structure anatomique quelconque.

3. Ecarteur suivant l'une des revendications **1. à 2. caractérisé en ce que** toutes les formes et dimensions peuvent être variables à condition qu'elles respectent les règles ergonomiques nécessaires à l'exposition parfaite d'une zone de l'anatomie humaine ou animale sans gêner le travail du chirurgien.

4. Ecarteur suivant l'une des revendications **1. à 3. caractérisé en ce qu'**il peut supporter sans se dégrader tout processus de stérilisation nécessaire à la réalisation optimale d'une procédure chirurgicale respectant les normes médicales de l'asepsie.

5. Ecarteur suivant l'une des revendications **1. à 4. caractérisé en ce qu'**il peut utiliser toute une variété d'instrumentations non spécifiques par l'intermédiaire d'un porteur **(16)** de forme et de dimension variable pour obtenir l'adaptation et la rétraction d'une structure anatomique quelconque vers un quelconque point hémisphérique Nord, supérieur, latéral ou externe par rapport au champ opératoire d'un sujet.

6. Ecarteur suivant l'une des revendications **1. à 5, caractérisé en ce qu'**il comprend une instrumentation spécifique **(15)** autorisant la saisie et la rétraction d'une structure anatomique quelconque vers l'anneau ou orbite équatoriale **(11)** et selon une inclinaison Sud ou inférieure grâce à l'axe de perforation de degré variable et à la localisation variable des orifices **(18)** des manches de l'instrumentation spécifique **(15)** variable en forme et en dimension.

7. Ecarteur suivant l'une des revendication **1. à 6. caractérisé en ce que** deux curseurs particuliers **(12')** diamétralement opposés autorisent le pivotement, la rotation et la fixation de l'anse ou orbite méridionale **(13)** selon un diamètre quelconque de l'anneau ou orbite équatoriale **(11)** tout en autorisant sa fixation selon une inclinaison variable de -90° à +90° par rapport à l'axe Nord-Sud perpendiculaire à l'anneau ou orbite équatoriale **(11).**

8. Ecarteur suivant l'une des revendications **1. à 7. caractérisé en ce qu'**il comprend des pièces amovibles **(17) et (17')** permettant un démontage intégral autorisant à la demande tout système d'accrochage et de rétraction respectant la fonctionnalité exigée et rendu possible par la possibilité de désolidarisation du bras horizontal **(9)** d'avec l'anneau ou orbite équatoriale **(11).**

9. Ecarteur selon l'une des revendications **1. à 9. caractérisé en ce qu'**il autorise de part son encombrement et ses possibilités de démontage, le nettoyage de toutes les pièces qui le composent avant d'entamer tout processus de stérilisation respectant les normes de l'asepsie chirurgicale.
